# EUROPEAN PATENT APPLICATION

(11) **EP 3 153 513 A1**
(43) Date of publication of application: **12.04.2017**
(21) Application number: 15188651.2
(22) Date of filing: 06.10.2015
(51) Int. Cl.: C07D 453/04, A61K 31/439

(54) **CRYSTALLINE ENCENICLINE HYDROCHLORIDE**

(71) Applicant: SANDOZ AG, 4056 Basel (CH)
(72) Inventor: The designation of the inventor has not yet been filed
(74) Representative: Kluschanzoff, Harald

(57) **Abstract**

The present invention relates to a crystalline form of encenicline hydrochloride and a process for its preparation. The invention also concerns a pharmaceutical composition comprising an effective amount of the crystalline form of encenicline hydrochloride and at least one pharmaceutically acceptable excipient. The pharmaceutical composition of the present invention can be used as medicament, in particular for treatment and/or prevention of CNS disorders associated with cognitive deficits such as Alzheimer's disease and schizophrenia.

## Description

### FIELD OF THE INVENTION

The present invention relates to a crystalline form of encenicline hydrochloride and a process for its preparation. The invention also concerns a pharmaceutical composition comprising an effective amount of the crystalline form of encenicline hydrochloride and at least one pharmaceutically acceptable excipient. The pharmaceutical composition of the present invention can be used as medicament, in particular for treatment and/or prevention of CNS disorders associated with cognitive deficits such as Alzheimer's disease and Schizophrenia.

### BACKGROUND OF THE INVENTION

Encenicline hydrochloride, chemically also designated (*R*)-7-chloro-*N-*(quinuclidin-3-yl)benzo[*b*]thiophene-2-carboxamide hydrochloride can be represented by the following chemical structure according to formula (A):

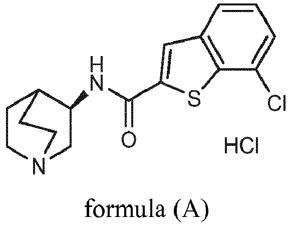

Acting as a partial, selective agonist of the alpha-7 nicotinic acetylcholine receptor (nAChR) encenicline is potentially useful for the treatment and/or prevention of cognitive deficits in Schizophrenia and Alzheimer's disease.

The active pharmaceutical ingredient encenicline hydrochloride and its synthesis are disclosed in WO 2003/055878 A1.

Furthermore, WO 2011/146511 A1 describes three crystalline forms of encenicline hydrochloride. Form I and form II are both monohydrates and form X is a form of low crystallinity, which may appear temporally during form I production.

A representative powder X-ray diffractogram of form X is displayed in figure 2 of WO 2011/146511 A1. The existence of broad and indistinct peaks as well as the low intensity of the reflections clearly indicate that form X is of low crystallinity. It is also explicitly mentioned in the application that form X is less stable than form I regardless of temperature. Hence, form X seems to be no viable form of encenicline hydrochloride for the use in a pharmaceutical formulation.

The two monohydrates, form I and form II also suffer from certain drawbacks, in particular they are both sensitive to temperature stress, which can become critical as particular pharmaceutical processing steps, such as milling or drying, usually involve the evolution of heat. For example, both monohydrates readily start to lose their water at moderate temperatures, whereas the dehydration process goes along with disruption of the crystal scaffold. In addition, both monohydrates exhibit low melting points, rendering them susceptive to melt-mediated polymorphic transformations upon excessive temperature stress.

The objective of the present invention was therefore the provision of a crystalline form of encenicline hydrochloride which would be more robust during pharmaceutical formulation steps where the generation of heat can be expected.

### SUMMARY OF THE INVENTION

The present invention relates to a new crystalline form of encenicline hydrochloride and a process for its preparation. The crystalline form of the invention is characterized by an extraordinary high melting point and high stability against temperature stress. It is therefore a physical form of encenicline hydrochloride with superior properties during pharmaceutical processing steps where the generation of heat can be expected. The crystalline form of encenicline hydrochloride of the present invention has the additional advantage that it enables the preparation of finished dosage forms comprising crystalline encenicline hydrochloride in the absence of amorphous encenicline hydrochloride, wherein the finished dosage form was prepared by a process including a process step where heat generation occurs.

### Definitions

In the context of the present invention the following definitions have the indicated meaning, unless explicitly stated otherwise:

As used herein "polymorph" refers to crystalline forms having the same chemical composition but different spatial arrangements of the molecules, atoms, and/or ions forming the crystal.

As used herein "solvate" refers to a crystalline form of a molecule, atom, and/or ions that further comprises molecules of a solvent or solvents incorporated into the crystalline lattice structure. The solvent molecules in the solvate may be present in a regular arrangement and/or a non-ordered arrangement. The solvate may comprise either a stoichiometric or nonstoichiometric amount of the solvent molecules. For example, a solvate with a nonstoichiometric amount of solvent molecules may result from partial loss of solvent from the solvate. When the solvent is water, the solvate is often referred to as a "hydrate". When the solvent is present in stoichiometric amount, the solvate may be referred to by adding greek numeral prefixes. For example, a hydrate may be referred to as monohydrate, dihydrate, trihydrate etc., depending on the water/encenicline monohydrochloride stoichiometry. The solvent content can be measured, for example, by GC, 'H-NMR or Karl-Fischer (KF) titration.

As used herein the term "room temperature" refers to temperatures in the range of from 20 to 30 °C.

The term "melting point" as used herein refers to the temperature at which a crystalline solid changes state from a solid to liquid.

As used herein, the term "substantially pure" with reference to a particular polymorphic form means that the polymorphic form includes less than 20%, preferably less than 10%, more preferably less than 5%, even more preferably less than 2%, most preferably less than 1% by weight of any other solid forms of the compound.

As used herein "amorphous" refers to a solid form of a molecule, atom, and/or ions that is not crystalline. An amorphous solid does not display a definitive X-ray diffraction pattern with reflections.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in peak positions and relative intensities of the peaks are to be taken into account. For example, a typical precision of the 2-theta values is in the range of ± 0.2° 2-theta. Thus, a diffraction peak that usually appears at 16.7° 2-theta for example can appear between 16.5° and 16.9° 2-theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

Crystalline encenicline hydrochloride may be referred to herein as being characterized by graphical data "as shown in" a figure. Such data include, for example, powder X-ray diffractograms (PXRDs) and differential scanning calorimetry (DSC) thermograms. The person skilled in the art understands that factors such as variations in instrument type, response and variations in sample directionality, sample concentration and sample purity may lead to small variations for such data when presented in graphical form, for example variations relating to the exact peak positions and intensities. However, a comparison of the graphical data in the figures herein with the graphical data generated for an unknown solid form and the confirmation that two sets of graphical data relate to the same crystal form is well within the knowledge of a person skilled in the art.

The term "form I" as used herein refers to the crystalline monohydrate of encenicline hydrochloride disclosed in WO 2011/146511 A1 which is characterized by having a PXRD comprising reflections at 2-Theta angles of (4.5 ± 0.2)°, (14.2 ± 0.2)°, (15.1 ± 0.2)°, (19.5 ± 0.2)° and (25.5 ± 0.2)°, when measured at room temperature with Cu-Kalpha_{1,2} radiation having a wavelength of 0.15419 nm.

The term "form II" as used herein refers to the crystalline monohydrate of encenicline hydrochloride disclosed in WO 2011/146511 A1 which is characterized by having a PXRD comprising reflections at 2-Theta angles of (4.5 ± 0.2)°, (14.3 ± 0.2)°, (21.2 ± 0.2)°, (21.4 ± 0.2)° and (25.0 ± 0.2)°, when measured at room temperature with Cu-Kalpha_{1,2} radiation having a wavelength of 0.15419 nm.

The term "solid form" as used herein refers to any crystalline and/or amorphous phase of a compound.

The term "about" as used herein means within 5%, more typically within 1% and most typically within 0.5% of the indicated value or range.

### BRIEF DESCRIPTION OF THE DRAWINGS

- Figure 1:: Representative powder X-ray diffractogram of the crystalline form of encenicline hydrochloride of the invention prepared according to example 1 herein. The x-axis shows the position in °2-Theta, the y-axis shows the number of counts in the detection unit of the diffractometer.
- Figure 2:: Unit cell of the crystalline form of encenicline hydrochloride of the invention. The atoms of the main component are drawn in capped-sticks style and the chloride ions are drawn as spheres.

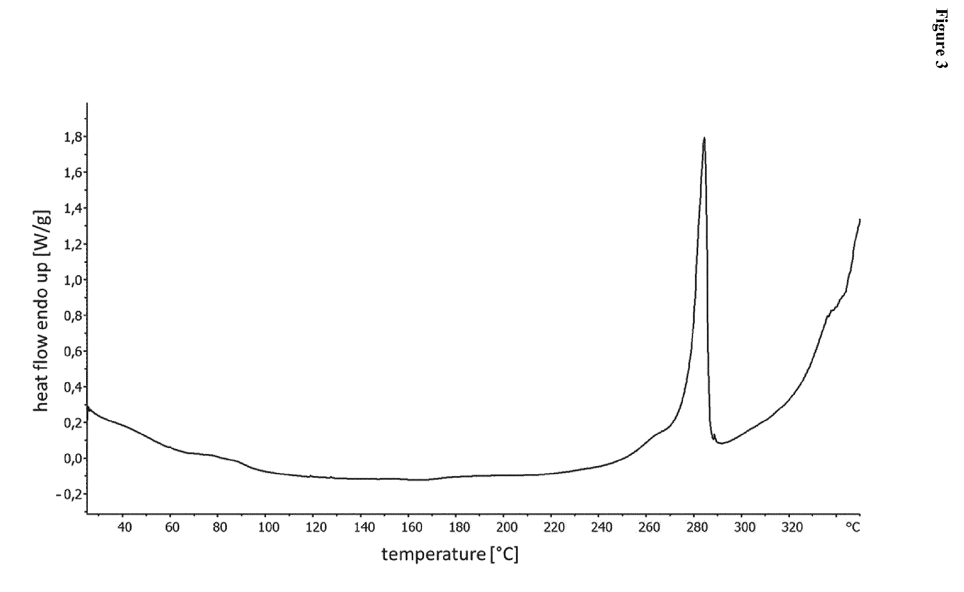

- Figure 3:: Representative differential scanning calorimetry thermogram of the crystalline form of encenicline hydrochloride of the invention prepared according to example 1 herein. The x-axis shows the temperature in °C, the y-axis the "heat flow endo-up" in W/g.

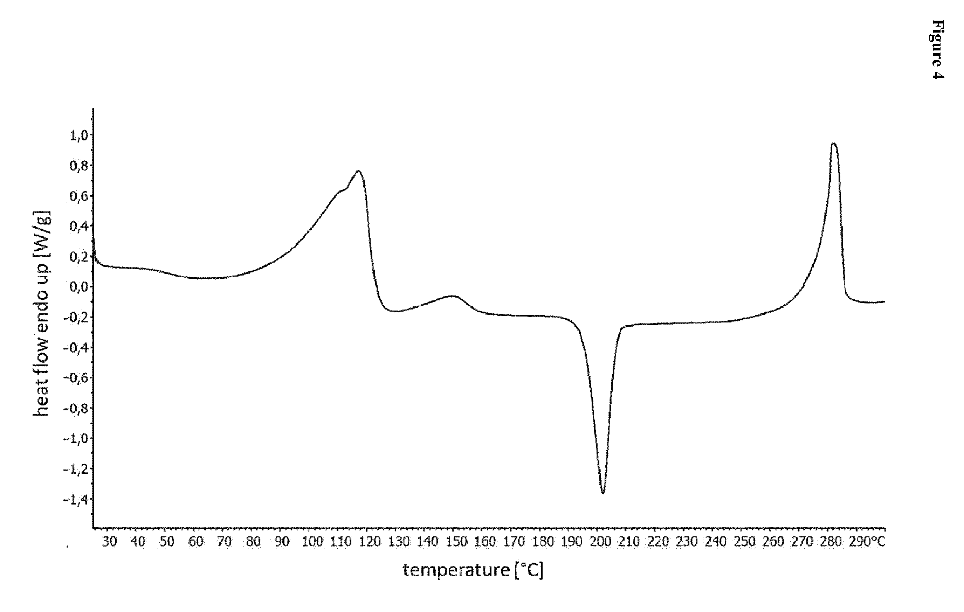

- Figure 4:: Representative differential scanning calorimetry thermogram of crystalline form I of encenicline hydrochloride monohydrate of WO 2011/146511 A1 prepared according to the procedure disclosed in example 2 of WO 2011/146511 A1. The x-axis shows the temperature in °C, the y-axis the "heat flow endo-up" in W/g.
- Figure 5:: Representative differential scanning calorimetry thermogram of crystalline form II of encenicline hydrochloride monohydrate of WO 2011/146511 A1 prepared according to the procedure disclosed in example 3 of WO 2011/146511 A1. The x-axis shows the temperature in °C, the y-axis the "heat flow endo-up" in W/g.

### DETAILED DESCRIPTION OF THE INVENTION

The invention is described below in further detail by embodiments, without being limited thereto.

The present invention relates to a new crystalline form of encenicline hydrochloride. Surprisingly, it has been found that crystalline encenicline hydrochloride of the present invention has an extraordinary high melting point. This property makes the crystalline encenicline hydrochloride of the present invention particularly useful for the preparation of such pharmaceutical formulations, where the preparation process involves a process step where heat is generated.

In a first aspect, the invention relates to a crystalline form of encenicline hydrochloride, in particular to a crystalline form of encenicline monohydrochloride. The crystalline form of encenicline monohydrochloride of the present invention is characterized by having a molar ratio of encenicline and hydrochloric acid in the range of from about 1.0: 0.8 to 1.2, preferably from about 1.0: 0.9 to 1.1 and most preferably the molar ratio is about 1.0: 1.0.

The crystalline form of encenicline hydrochloride of the present invention may be characterized by analytical methods well known in the field of the pharmaceutical industry for characterizing solids. Such methods comprise but are not limited to powder X-ray diffraction, single crystal X-ray diffraction and differential scanning calorimetry. It may be characterized by one of the aforementioned methods or by combining two or more of them. In particular, the crystalline encenicline hydrochloride, such as the crystalline monohydrochloride, of the present invention may be characterized by one of the following embodiments or by combining two or more of the following embodiments.

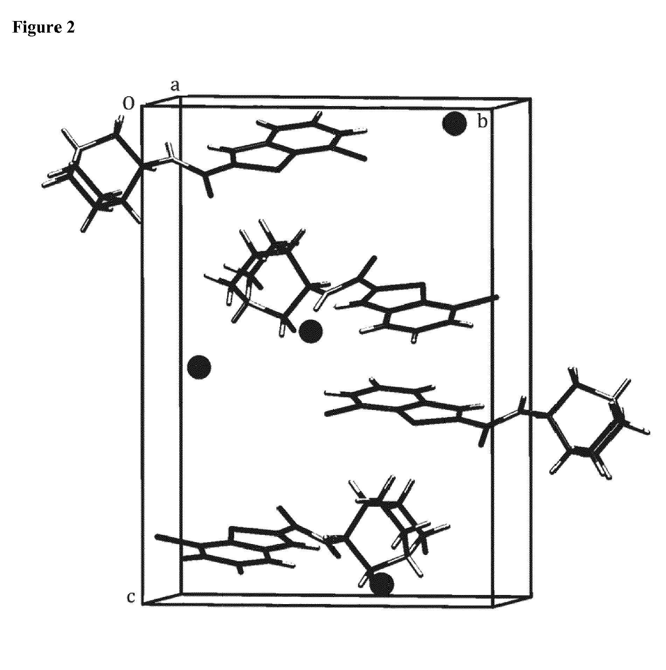

The crystalline form of the present invention can be clearly distinguished from form X of WO 2011/146511 A1 for example by its characteristic powder X-ray diffractogram. For example, the diffractogram of form X displayed in figure 2 of WO 2011/146511 A1 shows no reflections in the range of from about 10.0 to 14.0 ° 2-theta, whereas the crystalline form of the present invention *inter alia* exhibits reflections at 2-theta angles of (11.7 ± 0.2)°, (13.5 ± 0.2)° and (13.6 ± 0.2)°.

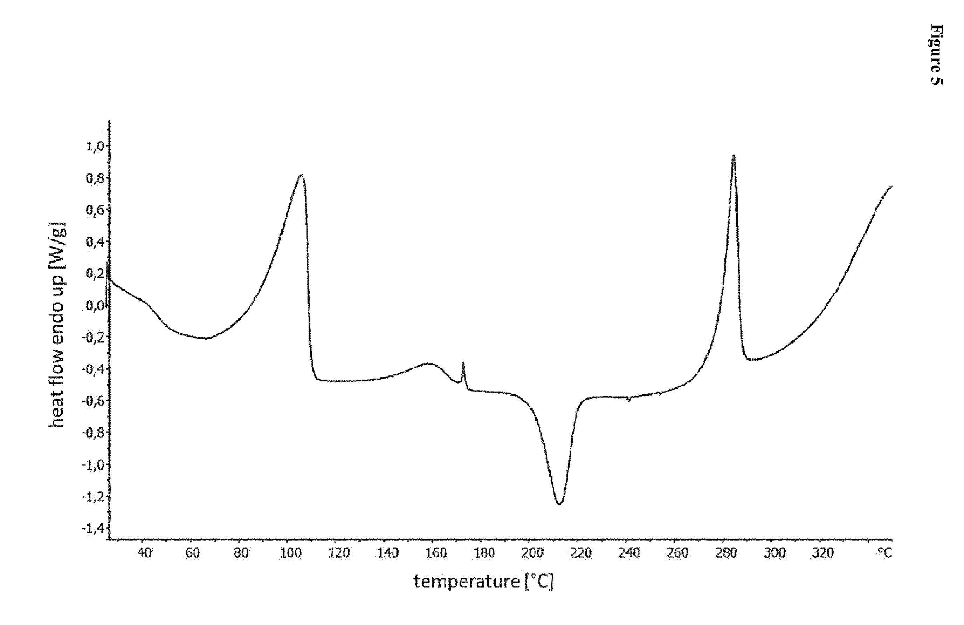

In addition, the powder X-ray diffractogram of the crystalline form of the present invention is also significantly different from the diffractograms of form I and II disclosed in figures 5 and 6 of WO 2011/146511 A1 respectively. In contrast to the powder X-ray diffractograms of form I and form II the diffractogram of the crystalline form of the present invention for example shows reflections at 2-theta angles of (8.2 ± 0.2)°, (9.6 ± 0.2)° and (11.7 ± 0.2)°. A comparison of the powder X-ray reflections of the crystalline form of the present invention, form I and form II in the range of from 2.0 to 20.0° 2-theta is displayed in table 1:

**Table 1: Powder X-ray reflection comparison of the different crystalline forms in the range of from 2.0 to 20.0° 2-theta**

| **Crystalline form of present invention** **[±0.2° 2-theta]** | **Form I** **(**WO 2011/146511 A1**)** **[±0.2° 2-theta]** | **Form II** **(**WO 2011/146511 A1**)** **[±0.2° 2-theta]** |
|---|---|---|
| | 4.5 | 4.5 |
| 8.2 | | |
| | 9.0 | 9.0 |
| 9.6 | | |
| 11.7 | | |
| 13.5 | | |
| 13.6 | | 13.6 |
| | 14.2 | |
| | | 14.3 |
| | 14.6 | |
| | 15.1 | |
| | | 15.6 |
| | 15.8 | |
| | | 16.5 |
| | 16.6 | |
| 16.7 | | |
| | 17.5 | |
| | | 18.1 |
| | 18.2 | 18.2 |
| | 18.4 | |
| | | 19.0 |
| 19.2 | | |
| | | 19.4 |
| | 19.5 | |
| 19.9 | | |

Therefore, the invention refers to a crystalline form of encenicline hydrochloride characterized by having a powder X-ray diffractogram comprising reflections at 2-theta angles of (13.6 ± 0.2)°, (16.7 ± 0.2)° and (19.9 ± 0.2)°, when measured at room temperature with Cu-Kalpha_{1,2} radiation having a wavelength of 0.15419 nm.

Alternatively or additionally, the invention refers to a crystalline form of encenicline hydrochloride characterized by having a powder X-ray diffractogram comprising reflections at 2-theta angles of (13.5 ± 0.2)°, (13.6 ± 0.2)°, (16.7 ± 0.2)° and (19.9 ± 0.2)°, when measured at room temperature with Cu-Kalpha_{1,2} radiation having a wavelength of 0.15419 nm.

Preferably, the invention refers to a crystalline form of encenicline hydrochloride characterized by having a powder X-ray diffractogram comprising reflections at 2-theta angles of (13.5 ± 0.2)°, (13.6 ± 0.2)°, (16.7 ± 0.2)°, (19.2 ± 0.2)° and (19.9 ± 0.2)°, when measured at room temperature with Cu-Kalpha_{1,2} radiation having a wavelength of 0.15419 nm.

More preferably, the invention refers to a crystalline form of encenicline hydrochloride characterized by having a powder X-ray diffractogram comprising reflections at 2-theta angles of (11.7 ± 0.2)°, (13.5 ± 0.2)°, (13.6 ± 0.2)° (16.7 ± 0.2)°, (19.2 ± 0.2)° and (19.9 ± 0.2)°, when measured at room temperature Cu-Kalpha_{1,2} radiation having a wavelength of 0.15419 nm.

Even more preferably, the invention refers to a crystalline form of encenicline hydrochloride characterized by having a powder X-ray diffractogram comprising reflections at 2-theta angles of (8.2 ± 0.2)°, (11.7 ± 0.2)°, (13.5 ± 0.2)°, (13.6 ± 0.2)°, (16.7 ± 0.2)°, (19.2 ± 0.2)° and (19.9 ± 0.2)°, when measured room temperature with Cu-Kalpha_{1,2} radiation having a wavelength of 0.15419 nm.

Particularly, the invention refers to a crystalline form of encenicline hydrochloride characterized by having a powder X-ray diffractogram comprising reflections at 2-theta angles of (8.2 ± 0.2)°, (9.6 ± 0.2)°, (11.7 ± 0.2)°, (13.5 ± 0.2)°, (13.6 ± 0.2)° (16.7 ± 0.2)°, (19.2 ± 0.2)°and (19.9 ± 0.2)°, when measured at room temperature with Cu-Kalpha_{1,2} radiation having a wavelength of 0.15419 nm.

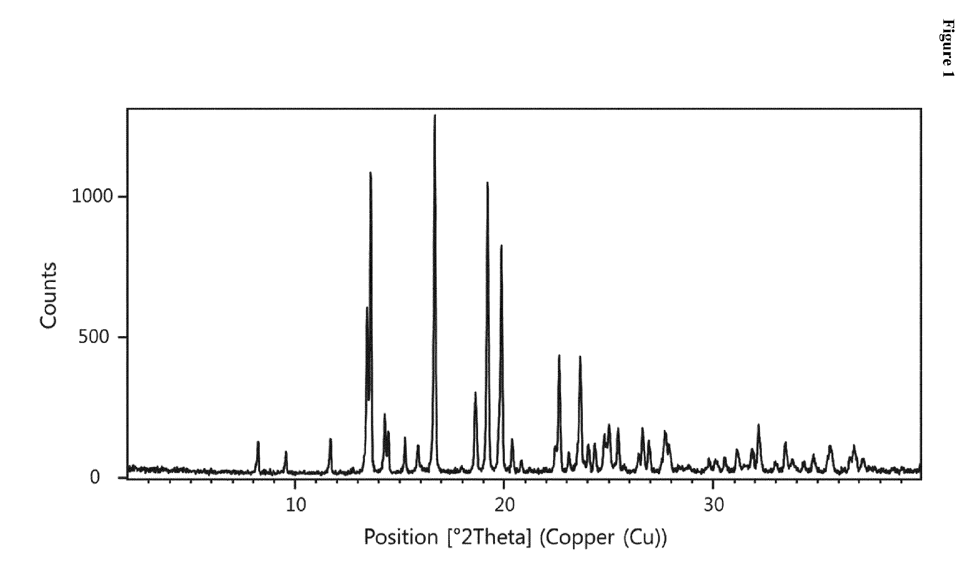

Alternatively, the invention refers to a crystalline form of encenicline hydrochloride characterized by having a powder X-ray diffractogram essentially the same as the one displayed in figure 1 of the present invention, when measured at room temperature with Cu-Kalpha_{1,2} radiation having a wavelength of 0.15419 nm.

Alternatively or additionally, the invention refers to a crystalline form of encenicline hydrochloride characterized by having the orthorhombic space group symmetry *P*2₁2₁2₁ and the following unit cell parameters as determined by an X-ray single crystal structure analysis at 173 K:
a = (7.46 ± 0.11) Angstrom;
b = (13.11 ± 0.20) Angstrom;
c = (18.41 ± 0.28) Angstrom;
alpha = 90.0°;
beta = 90.0°;
gamma = 90.0°;
Z = 4;

Alternatively or additionally, the invention relates to a crystalline form of encenicline hydrochloride characterized by having a differential scanning calorimetry curve showing a single endothermic signal, when measured at a heating rate of 10 K/min and starting from room temperature, wherein the single endothermic signal is at a temperature above about 200 °C, preferably above about 220 °C and most preferably above about 240 °C.

Preferably, the invention relates to a crystalline form of encenicline hydrochloride characterized by exhibiting a single melting point with an onset temperature above about 200 °C, preferably above about 220 °C and most preferably above about 240 °C, when measured with differential scanning calorimetry at a heating rate of 10 K/min and starting at room temperature.

In a further embodiment the invention relates to the crystalline form of encenicline hydrochloride of the present invention comprising less than 20 weight%, preferably less than 10 weight%, more preferably less than 5 weight% and most preferably less than 2 weight% of any other solid form of encenicline hydrochloride.

In a further aspect, the invention relates to a process for the preparation of the crystalline form of encenicline hydrochloride of the present invention comprising:
(i) providing a solid form of encenicline hydrochloride,
(ii) heating the solid form of encenicline hydrochloride of (i) in order to receive a melt and
(iii)allowing the melt obtained in (ii) to crystallize at a temperature in the range of from about 210 to 250 °C.

Examples of suitable solid forms of encenicline hydrochloride which may be applied in step (i) are form I and form II of WO 2011/146511 A1, which may be prepared according to the procedures disclosed in example 2 and example 3 of the same application respectively. Due to their low melting points form I and form II are especially suitable crystalline forms of encenicline hydrochloride for the production of the crystalline form of the present invention. Alternatively, amorphous encenicline hydrochloride can be used.

Melting in step (ii) is performed by, for example, heating form I and/or form II and/or amorphous encenicline hydrochloride for example above about 140 °C, preferably above about 160 °C, more preferably above about 180 °C and most preferably above about 200 °C for a period sufficient to receive a melt. In the subsequent step (iii) the obtained melt is annealed at a temperature in the range of from about 210 to 250 °C, preferably from about 220 to 250 °C, more preferably from about 230 to 250 °C and most preferably from about 240 to 250 °C for a period sufficient to receive crystals. Usually, crystals are obtained within a few minutes, for example within about 1 to 10 minutes. Once crystals are obtained they may optionally be cooled to room temperature.

In contrast to forms I and II of WO 2011/146511 A1 the crystalline form of the present invention exhibits an extraordinary high melting point and is thus especially stable against temperature stress. Table 2 summarizes the melting point onset temperatures of the different crystalline forms of encenicline hydrochloride determined by differential scanning calorimetry at a heating rate of 10 K/min.

**Table 2: Comparison of melting point onsets of the different crystalline forms at a heating rate of 10K/min**

| **Form** | **Onset temperature** |
|---|---|
| Crystalline form of present invention | 278 °C |
| Form I of WO 2011/146511 A1 | 133 °C |
| Form II of WO 2011/146511 A1 | 140 °C |

As can be seen from table 2 the crystalline form of the present invention clearly shows the highest melting point onset temperature. In addition, the differential scanning calorimetry curve of the crystalline form of the present invention shows no thermal event up to the melting point and therefore can be considered the most stable form of encenicline hydrochloride against temperature stress.

The crystals obtained according to the aforementioned procedure may be used as seed crystals, for example they may be added to a melt of encenicline hydrochloride or to a saturated solution of encenicline hydrochloride in a suitable solvent in order to promote crystallization of the crystalline form of the present invention.

Hence, in a further aspect the present invention relates to the use of the crystalline form of encenicline hydrochloride of the present invention as seed crystals in a process for the preparation of the crystalline form of encenicline hydrochloride of the present invention.

In a further aspect the invention relates to the use of the crystalline form of encenicline hydrochloride of the present invention for the preparation of a pharmaceutical composition.

An additional embodiment of the present invention concerns a pharmaceutical composition comprising an effective amount of the crystalline form of encenicline hydrochloride of the present invention and at least one pharmaceutically acceptable excipient.

For example, microcrystalline cellulose can be used as a diluent and binder, anhydrous calcium hydrogen phosphate as a diluent, croscarmellose sodium as a disintegrant, anhydrous colloidal silica as a glidant and magnesium stearate as a lubricant in the pharmaceutical composition of the present invention.

Crystalline encenicline hydrochloride of the present invention is preferably administered once a day at a dosage of 1 mg to 2 mg.

Furthermore, the invention relates to a pharmaceutical composition comprising an effective amount of the crystalline form of encenicline hydrochloride of the present invention and at least one pharmaceutically acceptable excipient for use as medicament.

Preferably, the invention relates to a pharmaceutical composition comprising an effective amount of the crystalline form of encenicline hydrochloride of the present invention and at least one pharmaceutically acceptable excipient for use in the treatment and/or prevention of cognitive disorders.

Finally, the invention also relates to a pharmaceutical composition comprising an effective amount of the crystalline form of encenicline hydrochloride of the present invention and at least one pharmaceutically acceptable excipient for use in the treatment of Alzheimer's disease, Cognition disorders, Schizoaffective disorder, Schizophrenia and/or Smoking withdrawal.

### Embodiment section

Aspects, advantageous features and preferred embodiments of the present invention are summarized in the following items:
1) A crystalline form of encenicline hydrochloride characterized by having a powder X-ray diffractogram comprising reflections at 2-theta angles of:
   (13.6 ± 0.2)°, (16.7 ± 0.2)° and (19.9 ± 0.2)°
   when measured at a temperature in the range of from 20 to 30 °C with Cu-Kalpha_{1,2} radiation having a wavelength of 0.15419 nm.
2) A crystalline form of encenicline hydrochloride characterized by having a powder X-ray diffractogram comprising reflections at 2-theta angles of:
   (13.5 ± 0.2)°, (13.6 ± 0.2)°, (16.7 ± 0.2)° and (19.9 ± 0.2)°
   when measured at a temperature in the range of from 20 to 30 °C with Cu-Kalpha_{1,2} radiation having a wavelength of 0.15419 nm.
3) A crystalline form of encenicline hydrochloride characterized by having a powder X-ray diffractogram comprising reflections at 2-theta angles of:
   (13.5 ± 0.2)°, (13.6 ± 0.2)°, (16.7 ± 0.2)°, (19.2 ± 0.2)° and (19.9 ± 0.2)°
   when measured at a temperature in the range of from 20 to 30 °C with Cu-Kalpha_{1,2} radiation having a wavelength of 0.15419 nm.
4) A crystalline form of encenicline hydrochloride characterized by having a powder X-ray diffractogram comprising reflections at 2-theta angles of:
   (11.7 ± 0.2)°, (13.5 ± 0.2)°, (13.6 ± 0.2)°, (16.7 ± 0.2)°, (19.2 ± 0.2)° and (19.9 ± 0.2)°,
   when measured at a temperature in the range of from 20 to 30 °C with Cu-Kalpha_{1,2} radiation having a wavelength of 0.15419 nm.
5) A crystalline form of encenicline hydrochloride characterized by having a powder X-ray diffractogram comprising reflections at 2-theta angles of:
   (8.2 ± 0.2)°, (11.7 ± 0.2)°, (13.5 ± 0.2)°, (13.6 ± 0.2)° (16.7 ± 0.2)°, (19.2 ± 0.2)°and (19.9 ± 0.2)°
   when measured at a temperature in the range of from 20 to 30 °C with Cu-Kalpha_{1,2} radiation having a wavelength of 0.15419 nm.
6) A crystalline form of encenicline hydrochloride characterized by having a powder X-ray diffractogram comprising reflections at 2-theta angles of:
   (8.2 ± 0.2)°, (9.6 ± 0.2)°, (11.7 ± 0.2)°, (13.5 ± 0.2)°, (13.6 ± 0.2)° (16.7 ± 0.2)°, (19.2 ± 0.2)°and (19.9 ± 0.2)°
   when measured at a temperature in the range of from 20 to 30 °C with Cu-Kalpha_{1,2} radiation having a wavelength of 0.15419 nm.
7) The crystalline form of encenicline hydrochloride according to any one of items 1 to 5, characterized by having a powder X-ray diffractogram not comprising any reflections at 2-theta angles in the range of from 4.0° to 5.0°.
8) The crystalline form of encenicline hydrochloride according to any one of items 1 to 6, characterized by having a powder X-ray diffractogram not comprising any reflections at 2-theta angles in the range of from 8.8° to 9.2°.
9) The crystalline form of encenicline hydrochloride according to any one of items 1 to 7, characterized by having a powder X-ray diffractogram not comprising any reflections at 2-theta angles of (4.5 ± 0.2)° and (9.0 ± 0.2)°.
10) The crystalline form of encenicline hydrochloride according to any one of items 1 to 8, characterized by having a powder X-ray diffractogram not comprising any reflections at 2-theta angles of (4.5 ± 0.2)°, (9.0 ± 0.2)° and (17.5 ± 0.2)°.
11) The crystalline form according to any one of items 1 to 9 characterized by having the orthorhombic space group symmetry *P*2₁2₁2₁ and the following unit cell parameters as determined by an X-ray single crystal structure analysis at a temperature of 173 K:
   a = (7.46 ± 0.11) Angstrom;
   b =(13.11 ± 0.20) Angstrom;
   c = (18.41 ± 0.28) Angstrom;
   alpha = 90.0°;
   beta = 90.0°;
   gamma = 90.0°;
   Z = 4;
12) The crystalline form according to any one of items 1 to 11 characterized by having a differential scanning calorimetry curve showing a single endothermic signal when measured at a heating rate of 10 K/min and starting from room temperature, wherein the single endothermic signal is at a temperature above 200 °C.
13) The crystalline form according to any one of items 1 to 12 characterized by exhibiting a single melting point with an onset temperature above 200 °C, when measured with differential scanning calorimetry at a heating rate of 10 K/min and starting at room temperature.
14) The crystalline form according to any one of items 1 to 13 comprising less than 20 weight% of any other solid form of encenicline hydrochloride based on the weight of the crystalline form as defined in any one of items 1 to 13.
15) The crystalline form according to any one of items 1 to 14 comprising less than 10 weight% of any other solid form of encenicline hydrochloride based on the weight of the crystalline form as defined in any one of items 1 to 13.
16) The crystalline form according to any one of items 1 to 15 comprising less than 5 weight% of any other solid form of encenicline hydrochloride based on the weight of the crystalline form as defined in any one of items 1 to 13.
17) The crystalline form according to any one of items 1 to 16 comprising less than 2 weight% of any other solid form of encenicline hydrochloride based on the weight of the crystalline form as defined in any one of items 1 to 13.
18) The crystalline form according to any one of items 1 to 17 comprising less than 2 weight% of amorphous encenicline hydrochloride.
19) A process for the preparation of crystalline encenicline hydrochloride according to any one of items 1 to 18 comprising:
   (i) providing a solid form of encenicline hydrochloride,
   (ii) heating the solid form of encenicline hydrochloride of (i) in order to receive a melt and
   (iii) allowing the melt obtained in (ii) to crystallize at a temperature in the range of from 210 to 250 °C.
20) The process of item 19, wherein the solid form of encenicline hydrochloride for step (i) is crystalline encenicline hydrochloride.
21) The process of item 20, wherein the crystalline encenicline hydrochloride is crystalline encenicline hydrochloride form I.
22) The process of item 20, wherein the crystalline encenicline hydrochloride is crystalline encenicline hydrochloride form II.
23) The process of item 19, wherein the solid form of encenicline hydrochloride for step (i) is amorphous encenicline hydrochloride.
24) Use of the crystals obtained by the process according to claim 19 as seed crystals in a process for the preparation of crystalline encenicline hydrochloride according to any one of items 1 to 18.
25) Use of crystalline encenicline hydrochloride according to any one of items 1 to 18 for the preparation of a pharmaceutical composition comprising encenicline hydrochloride.
26) A pharmaceutical composition comprising crystalline encenicline hydrochloride according to any one of items 1 to 18 and at least one pharmaceutically acceptable excipient.
27) The pharmaceutical composition according to item 26 for use as a medicament.
28) The pharmaceutical composition according to item 26 for use in the treatment and/or prevention of cognitive disorders.
29) The pharmaceutical composition according to item 26 for use in the treatment of Alzheimer's disease, Cognition disorders, Schizoaffective disorder, Schizophrenia and/or Smoking withdrawal.

The following non-limiting examples are illustrative for the disclosure.

### EXAMPLES

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-theta at ambient conditions. A typical precision of the 2-theta values is in the range of ± 0.2° 2-theta. Thus, the diffraction peak of form A that appears for example at 16.7° 2-theta can appear between 16.5 and 16.9° 2-theta on most X-ray diffractometers under standard conditions.

Intensity data for the crystal structure were collected with Cu-Kalpha_{1,2} radiation (wavelength: 0.15419 nm) on an Oxford Diffraction Gemini-R Ultra diffractometer at 173 K. The structure was solved using the direct methods procedure in SHELXS97 and refined by full-matrix least squares on F² using SHELXL97.

DSC was performed on a Mettler Polymer DSC R instrument. The samples (3.34 mg crystalline form of the present invention, 2.47 mg form I of WO 2011/146511 A1, 1.52 mg form II of WO 2011/146511 A1) were heated in a 40 microL aluminum pan with pierced aluminum lid either from 25 to 350 °C (in case of form A and form II) or from 25 to 300 °C (in case of form I) at a rate of 10 K/min. Nitrogen (purge rate 50 mL/min) was used as purge gas.

### Example 1: Preparation of the crystalline form of encenicline hydrochloride of the present invention starting from form I of WO 2011/146511 A1

Encenicline hydrochloride form I (53 mg, prepared according to the procedure disclosed in example 2 of WO 2011/146511 A1) was heated on a Kofler microscope at 30 K/min to a temperature of about 240 °C. The resulting melt was annealed at 240 °C until crystallization occurred (approximately 5 minutes). Finally, the crystals were allowed to cool to room temperature and scratched from the microscope slide.

Differential scanning calorimetry (10 K/min): single melting endotherm with an onset temperature of about 278 °C;

The powder X-ray diffractogram of the obtained material is displayed in figure 1 and a list of reflections and the corresponding relative intensities are provided in table 3. Powder X-ray diffraction confirmed the receipt of the crystalline form of the present invention.

**Table 3: Reflection list and relative intensities in the range of from 2.0 to 30.0° 2-theta of the crystalline form of encenicline hydrochloride of the present invention prepared according to example 1**

| Angle [± 0.2 °2-Theta] | Relative Intensity [%] | Angle [± 0.2 °2-Theta] | Relative Intensity [%] |
|---|---|---|---|
| 8.2 | 9 | 22.5 | 7 |
| 9.6 | 6 | 22.6 | 30 |
| 11.7 | 10 | 23.1 | 5 |
| 13.5 | 48 | 23.6 | 32 |
| 13.6 | 85 | 24.0 | 8 |
| 14.3 | 17 | 24.3 | 7 |
| 14.5 | 11 | 24.8 | 11 |
| 15.3 | 10 | 25.0 | 13 |
| 15.9 | 8 | 25.5 | 12 |
| 16.7 | 100 | 26.5 | 13 |
| 18.6 | 19 | 26.9 | 8 |
| 19.2 | 84 | 27.7 | 11 |
| 19.9 | 66 | 27.9 | 7 |
| 20.4 | 10 | 29.8 | 3 |
| 20.8 | 3 | | |

### Example 2: Preparation of the crystalline form of encenicline hydrochloride of the present invention starting from form II of WO 2011/146511 A1

Encenicline hydrochloride form II (25 mg, prepared according to the procedure disclosed in example 3 of WO 2011/146511 A1) was heated on a Kofler microscope at 30 K/min to a temperature of about 240 °C. The resulting melt was annealed at 240 °C until crystallization occured (approximately 5 minutes). Finally, the crystals were allowed to cool to room temperature and scratched from the microscope slide. Powder X-ray diffraction confirmed the receipt of the crystalline form of the present invention.

## Claims

1. A crystalline form of encenicline hydrochloride **characterized by** having a powder X-ray diffractogram comprising reflections at 2-theta angles of:
(13.6 ± 0.2)°, (16.7 ± 0.2)° and (19.9 ± 0.2)°
when measured at a temperature in the range of from 20 to 30 °C with Cu-Kalpha_{1,2} radiation having a wavelength of 0.15419 nm.

2. The crystalline form according to claim 1 **characterized by** having the orthorhombic space group symmetry *P*2₁2₁2₁ and the following unit cell parameters as determined by an X-ray single crystal structure analysis at a temperature of 173 K:
a = (7.46 ± 0.11) Angstrom;
b = (13.11 ± 0.20) Angstrom;
c = (18.41 ± 0.28) Angstrom;
alpha = 90.0°;
beta = 90.0°;
gamma = 90.0°;
Z = 4;

3. The crystalline form according to claim 1 or 2 **characterized by** having a differential scanning calorimetry curve showing a single endothermic signal when measured at a heating rate of 10 K/min and starting at a temperature in the range of from 20 to 30 °C, wherein the single endothermic signal is at a temperature above 200 °C.

4. The crystalline form according to any one of the preceding claims **characterized by** exhibiting a single melting point with an onset temperature above 200 °C, when measured with differential scanning calorimetry at a heating rate of 10 K/min and starting at a temperature in the range of from 20 to 30 °C.

5. The crystalline form according to any one of the preceding claims comprising less than 20 weight%, less than 10 weight%, less than 5 weight% or less than 2 weight% of any other solid form of encenicline hydrochloride based on the weight of the crystalline form as defined in any one of the preceding claims.

6. A process for the preparation of the crystalline form as defined in any one of the preceding claims comprising:
(i) providing a solid form of encenicline hydrochloride,
(ii) heating the solid form of encenicline hydrochloride of (i) in order to receive a melt and
(iii)allowing the melt obtained in (ii) to crystallize at a temperature in the range of from 210 to 250 °C.

7. Use of the crystals obtained in the process according to claim 6 as seed crystals in a process for the preparation of the crystalline form according to any one of claims 1 to 5.

8. Use of the crystalline form as defined in any one of claims 1 to 5 for the preparation of a pharmaceutical composition comprising encenicline hydrochloride.

9. A pharmaceutical composition comprising an effective amount of the crystalline form as defined in any one of claims 1 to 5 and at least one pharmaceutically acceptable excipient.

10. The pharmaceutical composition according to claim 9 for use as a medicament.

11. The pharmaceutical composition according to claim 9 for use in the treatment and/or prevention of cognitive disorders.

12. The pharmaceutical composition according to claim 9 for use in the treatment of Alzheimer's disease, Cognition disorders, Schizoaffective disorder, Schizophrenia and/or Smoking withdrawal.
